# EUROPÄISCHE PATENTSCHRIFT

(11) **EP 3 025 756 B1**
(45) Veröffentlichungstag und Bekanntmachung des Hinweises auf die Patenterteilung: **09.01.2019**
(21) Anmeldenummer: 15190500.7
(22) Anmeldetag: 20.10.2015
(51) Int. Cl.: A61N 1/37, A61N 1/08, A61N 1/375

(54) **IM AKTIVEN IMPLANTAT INTEGRIERTE ELEKTRODENVERLÄNGERUNG**
ELECTRODE EXTENSION INTEGRATED IN AN ACTIVE IMPLANT
PROLONGEMENT D'ELECTRODES INTEGRE DANS UN IMPLANT ACTIF

(30) Priorität: 27.11.2014 US 201462085249 P
(43) Veröffentlichungstag der Anmeldung: 01.06.2016
(73) Patentinhaber: BIOTRONIK SE & Co. KG, 12359 Berlin (DE)
(72) Erfinder: Rump, Jens, 12049 Berlin (DE)
(74) Vertreter: Galander, Marcus

(56) Entgegenhaltungen:
- US-A1- 2010 174 348
- US-A1- 2012 109 261

## Beschreibung

Die Erfindung betrifft ein permanent oder temporär implantierbares Gerät zur Verwendung mit einem langgestreckten elektrischen Leiter.

Langgestreckte elektrische Leiter sind beispielsweise in Elektrodenleitungen für die Elektrostimulation enthalten. Diese haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen. Deshalb können Herzschrittmacherpatienten heutzutage in der Regel nicht oder nur eingeschränkt in einem Kernspintomografen untersucht werden.

An implantierbaren Herzschrittmachern oder Defibrillatoren (im Folgenden auch gemeinsam als Herzstimulatoren oder IPG (implantable pulse generator) bezeichnet) sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ring-Elektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleiter bezeichnet. Solche Funktionsleiter sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleiter oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.
Die US-Patentanmeldung US 2010/0174348 A1 beschreibt eine implantierbare Elektrodenleitung, die mit einem Magnetresonanztomographen kompatibel ist. Diese Elektrodenleitung hat eine Mehrzahl gewundener Leiter, die als resonanter Filter wirken. In der US-Patentanmeldung US 2012/0109261 A1 werden Techniken beschrieben, mittels derer ein Implantat vor elektromagnetischen Störfeldern geschützt werden kann. Darüber hinaus wird dort eine Stichleitung als Filter in der Telemetrie-Einheit beschrieben.
Der Erfindung liegt die Aufgabe zugrunde, ein Gerät zu schaffen, welches das zuvor beschriebene Problem löst.
Erfindungsgemäß wird diese Aufgabe durch ein temporär oder permanent implantierbares medizinisches Gerät gelöst, das ein Gehäuse aufweist und mit wenigstens einem langgestreckten elektrischen Funktionsleiter für die Übertragung von Therapiesignalen oder Diagnosesignalen oder beidem verbunden oder zu verbinden ist, und wenigstens einem mit dem Funktionsleiter verbundenen Elektrodenpol, über den elektrischer Strom an im Benutzungsfall anliegendes Körpergewebe abgegeben oder mit dem elektrische Potentiale in im Benutzungsfall umgebenden Gewebe abgefühlt werden können oder beides.

Erfindungsgemäß weist das medizinische Gerät in dem Gehäuse eine elektrische Komponente als Leitungsverlängerung auf, die mit dem Funktionsleiter verbunden oder zu verbinden ist und die eine elektrische Länge aufweist, die wenigstens ein Viertel der Wellenlänge λ (Lambda) von elektromagnetischen Wellen im Radiofrequenzbereich wie sie in Kernspinntomographen auftreten, beträgt.

Durch Verwendung einer Verlängerung der Elektrodenleitung innerhalb des Gehäuses des implantierbaren medizinischen Gerätes wird eine Verringerung von unerwünschter RF-induzierter Erwärmung erreicht, ohne dass Veränderungen am Design der Elektrodenleitung notwendig sind. Dadurch, dass der für RF-induzierte Erwärmung empfindliche proximale Anteil der Elektrodenleitung durch das Gehäuse des Schrittmachers abgeschirmt wird, ist die Wirkung unabhängig von der Frequenz der eingestrahlten Welle und ist somit sowohl für 1.5 Tesla wie auch für 3 Tesla MRT wirksam.

Durch Verlängerung der Zuleitung innerhalb des Gehäuses des aktiven implantierbaren medizinischen Gerätes wird die eingekoppelte Energie am proximalen Ende der Elektrodenleitung so reduziert, dass am Punkt der Auskopplung elektrischer Signale (z. B. Stimulation oder Wahrnehmung) am distalen Ende die Amplitude elektromagnetischer Wellen möglichst gering ist.

Ein medizinisches Gerät, für das die Erfindung besonders relevant ist, ist ein Herzstimulator mit einer Elektrodenleitung, bei der die Funktionsleiter elektrische Leiter der Elektrodenleitung sind, wobei die Elektrodenleitung Elektrodenpole aufweist, welche über die Funktionsleiter mit dem Wellentransfermodul elektrisch verbunden sind.

Mittels der erfindungsgemäßen Leitungsverlängerung werden Nachteile bekannter Lösungen wie Bandstoppfilter, Tiefpassfilter, Shunt, Billabong oder Stichleitung vermieden. Solche Nachteile sind unter anderem: Filterelemente werden in den Elektrodenkörper integriert. Bewährte Designkonzepte können nicht mehr verwendet werden, so dass zwangsläufig eine Abweichung vom optimalen Elektrodendesign notwendig wird. Das Design wird bzgl. seiner physiologischen Eigenschaften geschwächt. Die Elektrode wird größer und steifer. Das Risiko von Gewebereizung und Perforation steigt. Darüber hinaus befinden sich Filterelemente im therapeutischen Pfad. Dadurch wird die Langlebigkeit der Konstruktion "gefährdet" oder eingeschränkt. Bandstoppfilter sind frequenzselektiv.

Gemäß einer Ausführungsvariante des erfindungsgemäßen implantierbaren medizinischen Gerätes kann das Gehäuse, beispielsweise eines an eine Elektrodenleitung angeschlossenen implantierbaren Herzstimulators, leitend ausgebildet sein oder einen Elektrodenpol aufweisen, über den im Benutzungsfall elektrischer Strom an umgebendes Körpergewebe abgegeben oder mit dem elektrische Potentiale in umgebenden Gewebe abgefühlt werden können oder beides.

Vorzugsweise weist das medizinische Gerät eine Komponente zur Impedanzanpassung zwischen dem Funktionsleiter und der Leitungsverlängerung (d.h. der elektrisch leitenden Komponente mit der elektrischen Länge > λ/4) auf, die ausgebildet ist, ein Vorhandensein starker elektromagnetischer Felder zu erfassen und im Falle des Vorhandenseins ein entsprechendes Ausgangssignal zu generieren, um Dämpfung einzustellen.

Gemäß einer Ausführungsvariante ist die Komponente zur Impedanzanpassung eine Stichleitung oder weist eine solche auf.

Gemäß einer Ausführungsvariante weist die Komponente zur Impedanzanpassung zusätzlich oder alternativ einen Balun auf.

Gemäß einer weiteren Ausführungsvariante weist die Komponente zur Impedanzanpassung zusätzlich oder alternativ eine Kapazität auf.

Gemäß einer weiteren Ausführungsvariante weist die Komponente zur Impedanzanpassung zusätzlich oder alternativ eine Induktivität auf.

Die Komponente zur Impedanzanpassung kann zusätzlich oder alternativ auch eine Kombination aus Induktivität und Kapazität sein oder eine solche Kombination aufweisen.

Vorzugsweise ist die elektrische Komponente, deren elektrische Länge mindestens ein Viertel der Wellenlänge λ von elektromagnetischen Wellen im Radiofrequenzbereich entspricht - also die Leitungsverlängerung -, entlang ihrer Länge isoliert.

Zusätzlich oder alternativ kann die elektrische Komponente, deren elektrische Länge mindestens ein Viertel der Wellenlänge λ von elektromagnetischen Wellen im Radiofrequenzbereich entspricht, Kontakt mit einem Medium haben, das ganz oder abschnittsweise aus einem der folgenden Werkstoffe oder einer Kombination der folgenden Werkstoffe besteht: Glas, Bariumtitanat, Polyethylen, Siliziumoxid, Siliziumkarbid, Keramik, Piezowerkstoffe, Aluminiumoxid, Titanoxid, Tantaloxid, Halbleiterwerkstoffe, Kunststoffe mit Beimengungen der oben genannten Werkstoffe.

Gemäß einer weiteren zusätzlichen oder alternativen Ausführungsvariante weist die elektrische Komponente, deren elektrische Länge mindestens ein Viertel der Wellenlänge λ von elektromagnetischen Wellen im Radiofrequenzbereich entspricht, einen Verlängerungsleiter auf, der als eine der Ausführungen oder einer Kombination: Draht/Band, platinierter Draht/Band, gewendelter Draht/Band, Litze, Seil, gedruckter Leiter, geschnittener Leiter, geätzter Leiter, galvanisch abgeschiedener Leiter, geschlitzte leitfähige Fläche realisiert ist.

Besonders bevorzugt ist ein batteriebetriebenes elektronisches Implantat mit einem elektrisch leitfähigen Gehäuse oder einem Gehäuse mit mindestens einem Elektrodenpol, elektrischen Funktionsleitern, die Elektrodenpole mit elektrischen Durchführungen des Implantats verbinden, um elektrische Signale von dem jeweiligen Elektrodenpol ins Implantat leiten zu können.

Die Erfindung soll nun anhand von Ausführungsbeispielen mit Bezug auf die Figuren näher erläutert werden. Die Figuren zeigen Folgendes:
- Fig. 1: zeigt als implantierbare medizinische Geräte einen implantierbaren Herzstimulator 10 und eine daran angeschlossene implantierbare Elektrodenleitung 20.
- Fig. 2: illustriert die Superposition elektromagnetischer Wellen in einer Elektrodenleitung.
- Fig. 3: illustriert die Amplituden äußerer Wechselmagnetfelder entlang implantierter Elektrodenleitungen.
- Fig. 4: zeigt in stark schematisierter Darstellung einen herkömmlichen Herzstimulator mit einer Filterdurchführung.
- Fig. 5: zeigt in stark schematisierter Darstellung einen erfindungsgemäßen Herzstimulator mit einer Leitungsverlängerung.

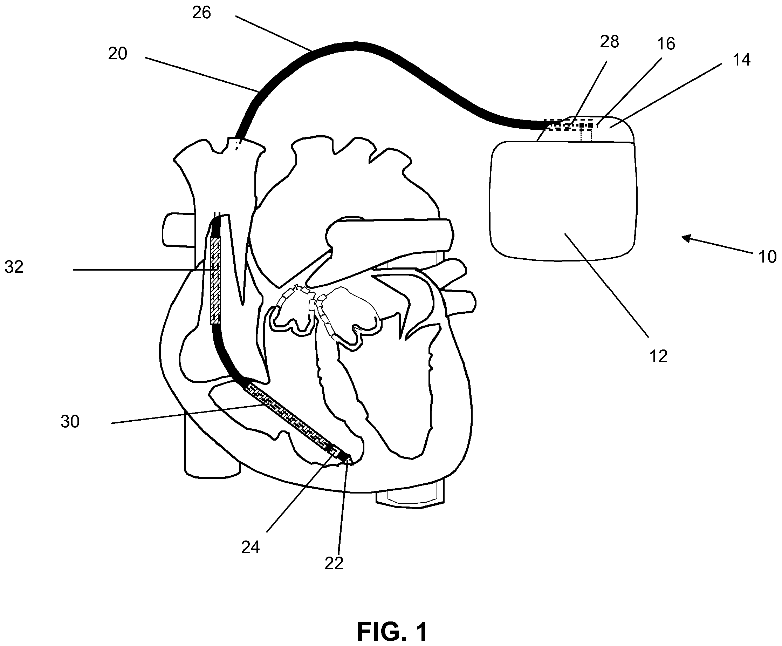

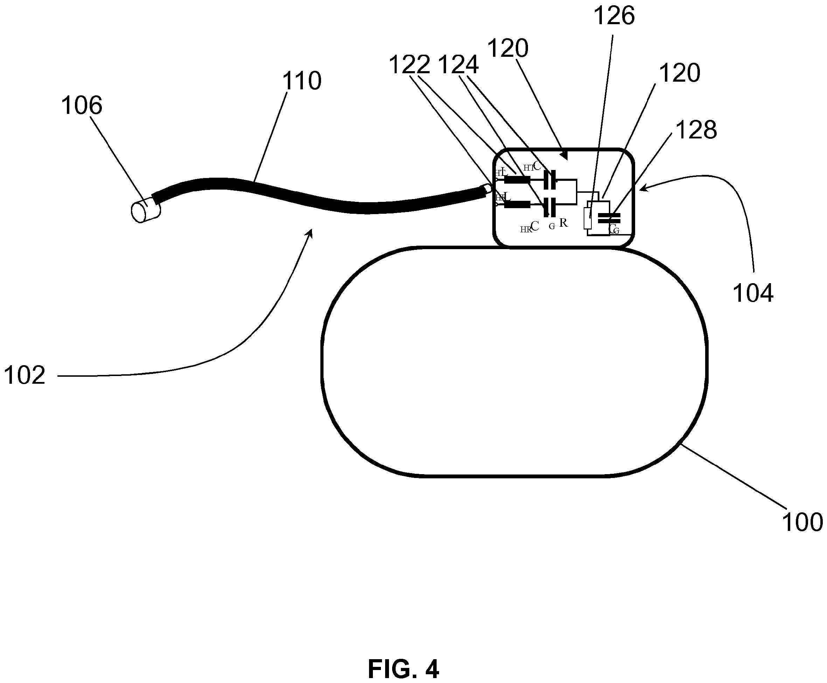

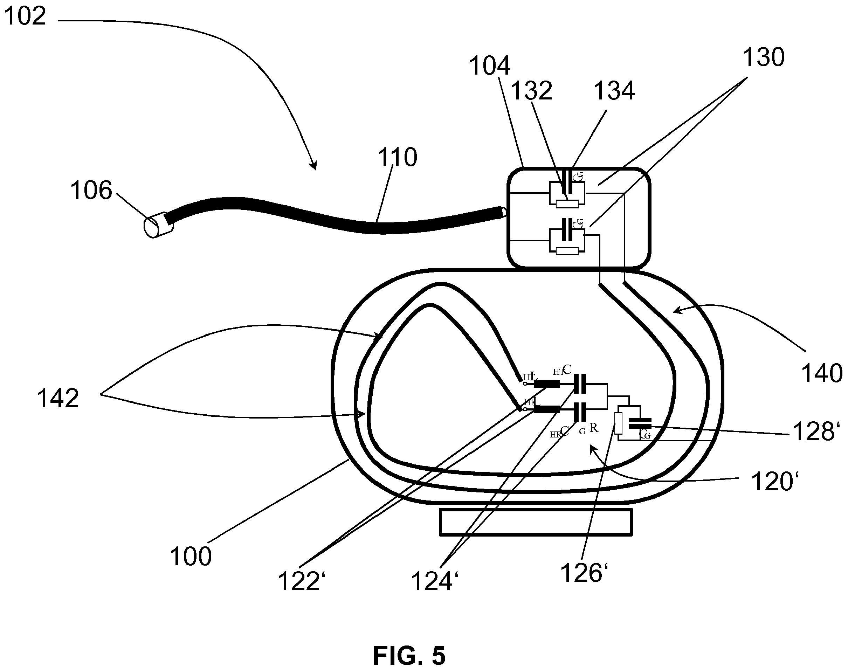

Figur 1 zeigt ein implantierbares aktives medizinisches Gerät in Form eines implantierbaren Herzstimulators 10. Erfindungsgemäße Details sind in Figuren 4 und 5 dargestellt.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren 10 besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordnete Elektronik verbunden sind.

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ring-Elektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Figur 1 zeigt, wie sich die Elektrodenpole, also die Tip-Elektrode 22 und die Ring-Elektrode 24, im Anwendungsfall die Elektrodenleitung 20, im Apex eines rechten Ventrikels eines Herzens befinden.

Sowohl die Tip-Elektrode 22 als auch die Ring-Elektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegenden helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

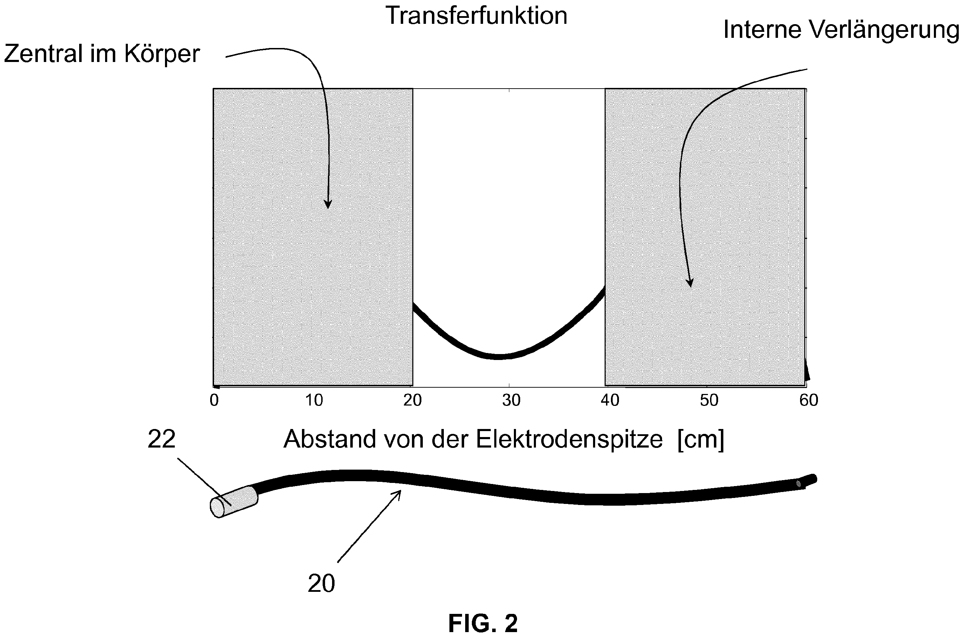

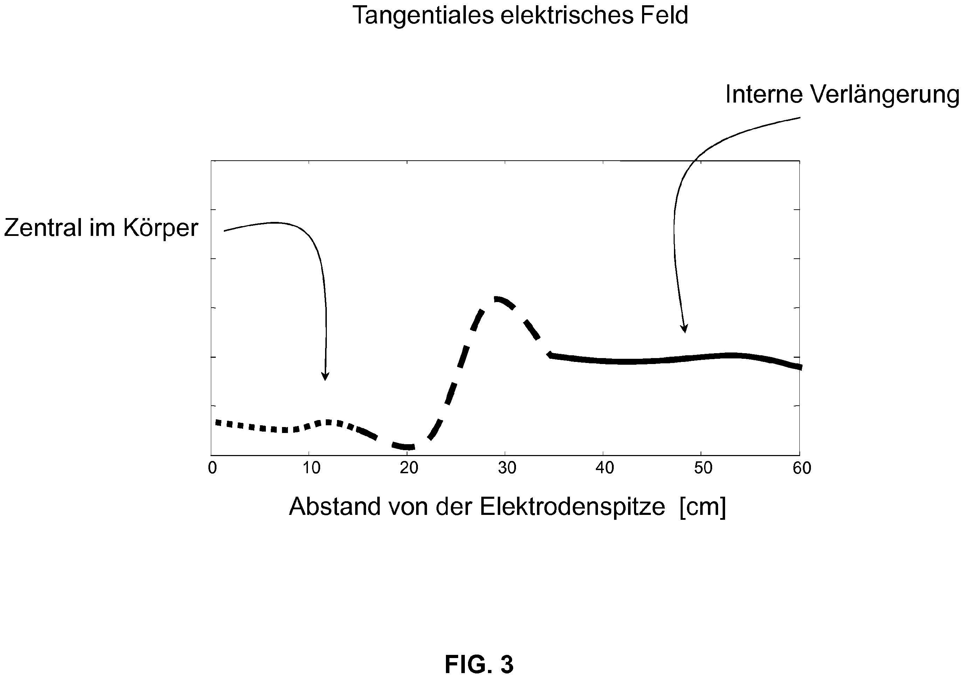

Figuren 2 und 3 illustrieren, wie ein äußeres elektromagnetisches Feld zu einer Erwärmung von Körpergewebe in der Nähe des distalen Endes einer Elektrodenleitung wie der Elektrodenleitung 20 führen kann.

Für die Einkopplung elektromagnetischer Wellen in langgestreckten elektrischen Leitern wie dem Leiter 26 der Elektrodenleitung 20 gilt das Superpositionsprinzip von Elementarwellen entlang des Leiters. Die Amplitude der Auskopplung ergibt sich aus der konstruktiven bzw. destruktiven Superposition der Wellen am Ort der Auskopplung. Da es sich bei implantierbaren Leitern um ein stark gedämpftes System handelt, ergibt sich bezüglich der Einkopplung elektromagnetischer Wellen am Ende des elektrischen Leiters folgendes: Eine Einkopplung in der Nähe sowohl des einen als auch des anderen Endes des elektrischen Leiters bewirkt durch konstruktive Überlagerung direkter und reflektierter Wellen Maxima für die Auskopplung (siehe Figur 2). Diese Maxima sind wegen der Dämpfung auch bei nicht resonanten Leitern zu beobachten. Betrachtet man die Lage der Enden nach Implantation eines langgestreckten Leiters und eines aktiven implantierbaren medizinischen Gerätes, so befindet sich ein Ende des Leiters innerhalb des Körpers mit einem größeren Abstand zur Körperoberfläche (distales Ende des Leiters bzw. der Elektrodenleitung) und ein anderes Ende in Kontakt mit dem aktiven implantierbaren medizinischen Gerät (proximales Ende des Leiters bzw. der Elektrodenleitung) in der Nähe der Körperoberfläche.

Im Bereich des distalen Endes ist die Amplitude der eingestrahlten Welle aufgrund der Dämpfung im Körper stark verringert und trägt damit nur reduziert zur Amplitude der Auskopplung am Elektrodenende bei (siehe Figur 3).

Das proximale Ende hingegen befindet sich nahe der Körperoberfläche im Schulterbereich und ist damit einem stärkeren externen Feld ausgesetzt.

Figur 4 zeigt das Anschlussgehäuse 104 und die elektrischen Komponenten einer Filterdurchführung eines herkömmlichen Herzschrittmachers. im Unterschied hierzu zeigt Figur 5 in schematischer Darstellung ein Beispiel eines Herzschrittmachers mit einer erfindungsgemäßen Leitungsverlängerung mit einer elektrischen Komponente 120, deren elektrische Länge mindestens ein Viertel der Wellenlänge λ von elektromagnetischen Wellen im Radiofrequenzbereich entspricht.

Das medizinische Gerät besitzt ein elektrisch leitendes Gehäuse 100, das dem Gehäuse 12 aus Figur 1 entspricht. An das Gehäuse 100 angeschlossen ist eine Elektrodenleitung 102, die eine Tip-Elektrode 106 aufweist. Die Tip-Elektrode 106 bildet einen Elektrodenpol. Der Elektrodenpol ist über eine Zuleitung 110 mit einer Elektronik im Inneren des Gehäuses 100 verbunden. Die Zuleitung 110 bildet einen Funktionsleiter.

Die Zuleitung 110 (Funktionsleiter) ist über einen in Figuren 4 und 5 nicht dargestellten Stecker geführt.

Bei dem Stand der Technik gemäß Figur 4 schließt sich an den Stecker eine Filterdurchführung mit einer Anschlusselektronik 120 an, an die wiederum die Herzschrittmacherelektronik (nicht dargestellt) im Inneren des Gehäuses 100 angeschlossen ist.

Die Anschlusselektronik 120 dient u.a. der Filterung der Signale und wird beispielsweise von zwei einander parallel geschalteten Serienschaltung einer Spule 122 und eines Kondensators 124 gebildet, an die sich wiederum eine Parallelschaltung eines ohmschen Widerstands 126 und eines weiteren Kondensators 128 anschließt. Die Anschlusselektronik ist in dem Anschlussgehäuse 104 angeordnet.

Gemäß dem Ausführungsbeispiel in Figur 5 sind in dem Anschlussgehäuse 104 Komponenten 130 zur Impedanzanpassung angeordnet, die jeweils von einem Ohmschen Widerstand 132 und einem dazu parallel geschalteten Kondensator 134 gebildet sind. Der Funktionsleiter 110 oder die Funktionsleiter 110 sind über die Komponente 130 zur Impedanzanpassung mit der Leitungsverlängerung 140 verbunden, die zwei Verlängerungsleiter 142 aufweist, die in dem Gehäuse 100 des Herzschrittmachers angeordnet sind und an ihrem proximalen, vom Anschlussgehäuse 104 entfernten Ende mit einer Anschlusselektronik 120' verbunden sind. Die Anschlusselektronik 120' in Figur 5 entspricht im Wesentlichen derjenigen aus Figur 4, nur ist sie nicht im Anschlussgehäuse 104, sondern im Gehäuse 100 angeordnet.

Auf diese Weise ergeben sich zwei Serienschaltungen, die eingangsseitig jeweils Komponenten 130 zur Impedanzanpassung aufweisen, daran anschließend jeweils einen Verlängerungsleiter 142, der mit einer Spule (Induktivität) 122' und einem Kondensator (Kapazität) 1234' abgeschlossen ist. Diese beiden Serienschaltungen sind einander parallel geschaltet, das heißt die beiden Kondensatoren 124' sind ausgangsseitig miteinander verbunden und ihrerseits wieder gemeinsam mit einer Parallelschaltung aus einem Ohmschen Widerstand 126' und einem Kondensator 128' verbunden.

Die elektrische Komponente 140 hat eine elektrische Länge, die mindestens ein Viertel der Wellenlänge λ von elektromagnetischen Wellen im Radiofrequenzbereich entspricht und die hier auch als Leitungsverlängerung bezeichnet wird.

Gegenüber einem herkömmlichen Herzschrittmacher wird bei dem erfindungsgemäßen Herzschrittmacher das Anschlussgehäuse (Header) ausgetauscht durch eine einfache Steckverbindung, vorzugsweise ergänzt durch die Komponenten 130 zur Impedanzanpassung an die Elektrodenleitung um Reflexionen zu minimieren.

Als Leitungsverlängerung 140 innerhalb des Schrittmachers werden (vorzugsweise niederohmige) Seile/Drähte 20cm entlang des Gehäuses 100 geführt und somit die elektrische Länge des Funktionsleiters 110 der Elektrodenleitung 102 entsprechend verlängert. Die Verlängerung entspricht in etwa einer zweifachen Wicklung der Elektrodenleitung um den Herzschrittmacher. Untersuchungen innerhalb eines RF-Resonators haben ergeben, dass zusätzliche Wicklungen von Elektrodenleitungen um den Herzschrittmacher zu einer deutlichen Reduzierung RF-induzierter Erwärmung führt, da der Bereich der Wicklungen teilweise durch das metallische Gehäuse des Herzschrittmachers abgeschirmt wird. Die eigentliche Anschlussstelle der Elektrodenleitung an die Funktionen des aktiven implantierbaren medizinisches Gerätes - hier: des Herzschrittmachers - befindet sich innerhalb des Gehäuses des Herzschrittmachers. Eine kapazitive Kopplung der Seile/Drähte an das Gehäuse des Herzschrittmachers ist eine zusätzliche Möglichkeit, eingekoppelte Energie an das umgebende Gewebe abzugeben ohne das Gewebe durch Wärmeeinwirkung zu schädigen.

## Patentansprüche

1. Temporär oder permanent implantierbares medizinisches Gerät, das ein Gehäuse (12, 100) umfasst und das mit wenigstens einem langgestreckten elektrischen Funktionsleiter für die Übertragung von Therapiesignalen oder Diagnosesignalen oder beidem verbunden oder zu verbinden ist, und wenigstens einem mit dem Funktionsleiter verbundenen Elektrodenpol (30, 32), über den elektrischer Strom an im Benutzungsfall umgebendes Körpergewebe abgegeben oder mit dem elektrische Potentiale in im Benutzungsfall umgebenden Gewebe abgefühlt werden können oder beides, wobei das medizinische Gerät mindestens eine elektrische Komponente aufweist, die sich innerhalb des Gehäuses (12, 100) befindet und deren elektrische Länge mindestens ein Viertel der Wellenlänge λ von elektromagnetischen Wellen im Radiofrequenzbereich, die in Kernspintomographen bei 1,5 T oder 3 T auftreten, entspricht, wobei der Funktionsleiter ein elektrischer Leiter (26) einer Elektrodenleitung (20, 102) ist, die wenigstens einen Elektrodenpol (30; 32) aufweist, welcher über den Funktionsleiter mit der innerhalb des Gehäuses (12, 100) angeordneten elektrischen Komponente, deren elektrische Länge mindestens ein Viertel der Wellenlänge λ von elektromagnetischen Wellen im Radiofrequenzbereich die in Kernspintomographen bei 1,5 T oder 3 T auftreten entspricht, elektrisch verbunden ist.

2. Medizinisches Gerät nach Anspruch 1, **dadurch gekennzeichnet, dass** das medizinische Gerät ein Gehäuse (12, 100) besitzt, welches elektrisch leitend ausgebildet ist oder einen Elektrodenpol (30, 32) aufweist, über den im Benutzungsfall elektrischer Strom an umgebendes Körpergewebe abgegeben oder mit dem elektrische Potentiale in umgebenden Gewebe abgefühlt werden können oder beides.

3. Medizinisches Gerät nach wenigstens einem der Ansprüche 1 oder 2, **dadurch gekennzeichnet, dass** das medizinische Gerät eine Komponente (130) zur Impedanzanpassung zwischen dem Funktionsleiter (110) und der elektrisch leitenden Komponente (140) mit der elektrischen Länge > λ/4 aufweist, die ausgebildet ist, die Impedanzen des Funktionsleiters (110) und der elektrisch leitenden Komponente (140) mit der elektrischen Länge > λ/4 aneinander anzupassen.

4. Medizinisches Gerät nach Anspruch 3, **dadurch gekennzeichnet, dass** die Komponente zur Impedanzanpassung (130) eine Stichleitung ist oder eine solche aufweist.

5. Medizinisches Gerät nach Anspruch 3 oder 4, **dadurch gekennzeichnet, dass** die Komponente zur Impedanzanpassung (130) ein Balun ist oder einen solchen aufweist.

6. Medizinisches Gerät nach wenigstens einem der Ansprüche 3 bis 5, **dadurch gekennzeichnet, dass** die Komponente (130) zur Impedanzanpassung eine Kapazität (134) ist oder eine solche aufweist.

7. Medizinisches Gerät nach wenigstens einem der Ansprüche 3 bis 6, **dadurch gekennzeichnet, dass** die Komponente (130) zur Impedanzanpassung eine Induktivität ist oder eine solche aufweist.

8. Medizinisches Gerät nach wenigstens einem der Ansprüche 3 bis 7, **dadurch gekennzeichnet, dass** die Komponente zur Impedanzanpassung (130) eine Kombination aus Induktivität und Kapazität ist oder eine solche Kombination aufweist.

9. Medizinisches Gerät nach wenigstens einem der Ansprüche 1 bis 8, **dadurch gekennzeichnet, dass** die elektrische Komponente (140), deren elektrische Länge mindestens ein Viertel der Wellenlänge λ von elektromagnetischen Wellen im Radiofrequenzbereich entspricht, entlang ihrer Länge isoliert ist.

10. Medizinisches Gerät nach wenigstens einem der Ansprüche 1 bis 9, **dadurch gekennzeichnet, dass** die elektrische Komponente, (140) deren elektrische Länge mindestens ein Viertel der Wellenlänge λ von elektromagnetischen Wellen im Radiofrequenzbereich entspricht, Kontakt mit einem Medium hat, das ganz oder abschnittsweise aus einem der folgenden Werkstoffe oder einer Kombination der folgenden Werkstoffe besteht: Glas, Bariumtitanat, Polyethylen, Siliziumoxid, Siliziumkarbid, Keramik, Piezowerkstoffe, Aluminiumoxid, Titanoxid, Tantaloxid, Halbleiterwerkstoffe, Kunststoffe mit Beimengungen der oben genannten Werkstoffe.

11. Medizinisches Gerät nach wenigstens einem der Ansprüche 1 bis 10, **dadurch gekennzeichnet, dass** die elektrische Komponente (140), deren elektrische Länge mindestens ein Viertel der Wellenlänge λ von elektromagnetischen Wellen im Radiofrequenzbereich entspricht, einen Verlängerungsleiter (142) aufweist, der als eine der Ausführungen oder einer Kombination: Draht/Band, platinierter Draht/Band, gewendelter Draht/Band, Litze, Seil, gedruckter Leiter, geschnittener Leiter, geätzter Leiter, galvanisch abgeschiedener Leiter, geschlitzte leitfähige Fläche realisiert ist.

## Claims

1. A temporarily or permanently implantable medical device, which comprises a housing (12, 100) and which is connected or is to be connected to at least one elongate electrical function conductor for the transmission of therapy signals or diagnosis signals or both, and at least one electrode pole (30, 32) connected to the function conductor, via which electrode pole electric current is delivered to surrounding body tissue during use or with which electrode pole electrical potentials can be sensed in surrounding during use, or both, wherein the medical device has at least one electrical component, which is located within the housing (12, 100) and of which the electric length corresponds to at least a quarter of the wavelength λ of electromagnetic waves in the radio frequency range, which occur in 1.5 T or 3 T MRI scanners, wherein the function conductor is an electrical conductor (26) of an electrode line (20, 102), which has at least one electrode pole (30; 32), which is electrically connected via the function conductor to the electrical component arranged within the housing (12, 100) and of which the electric length corresponds to at least a quarter of the wavelength λ of electromagnetic waves in the radio frequency range occurring in 1.5 T or 3 T MRI scanners.

2. The medical device according to claim 1, **characterised in that** the medical device has a housing (12, 100), which is electrically conductive, or has an electrode pole (30, 32), via which, during use, electric current is delivered to surrounding body tissue or with which electrical potentials can be sensed in surrounding tissue, or both.

3. The medical device according to at least one of claims 1 or 2, **characterised in that** the medical device has a component (130) for impedance matching between the function conductor (110) and the electrically conductive component (140) with the electric length > λ/4, which component is configured to match to one another the impedances of the function conductor (110) and the electrically conductive component (140) with the electric length > λ/4.

4. The medical device according to claim 3, **characterised in that** the component for impedance matching (130) is a spur line or has such a line.

5. The medical device according to claim 3 or 4, **characterised in that** the component for impedance matching (130) is a balun or has a balun.

6. The medical device according to at least one of claims 3 to 5, **characterised in that** the component (130) for impedance matching is a capacitor (134) or has a capacitor.

7. The medical device according to at least one of claims 3 to 6, **characterised in that** the component (130) for impedance matching is an inductor or has an inductor.

8. The medical device according to at least one of claims 3 to 7, **characterised in that** the component for impedance matching (130) is a combination of inductor and capacitor or has such a combination.

9. The medical device according to at least one of claims 1 to 8, **characterised in that** the electrical component (140), of which the electric length corresponds to at least a quarter of the wavelength λ of electromagnetic waves in the radio frequency range, is insulated along the length thereof.

10. The medical device according to at least one of claims 1 to 9, **characterised in that** the electrical component (140), of which the electric length corresponds to at least a quarter of the wavelength λ of electromagnetic waves in the radio frequency range, has contact with a medium that consists fully or in portions of one of the following materials or a combination of the following materials: glass, barium titanate, polyethylene, silicon oxide, silicon carbide, ceramic, piezo materials, alumina, titanium oxide, tantalum oxide, semiconductor materials, plastics with additions of the above-mentioned materials.

11. The medical device according to at least one of claims 1 to 10, **characterised in that** the electrical component (140), of which the electric length corresponds to at least a quarter of the wavelength λ of electromagnetic waves in the radio frequency range, has an extension conductor (142), which is formed as one of the following embodiments or a combination thereof: wire/band, platinum-plated wire/band, wound wire/band, stranded wire, cable, printed conductor, cut conductor, etched conductor, galvanically deposited conductor, slitted conductive surface

## Revendications

1. Appareil médical implantable de manière temporaire ou permanente qui comprend un boîtier (12, 100) et qui est relié ou est à relier avec au moins un conducteur fonctionnel électrique allongé pour la transmission de signaux thérapeutiques ou de signaux de diagnostic, ou des deux, et au moins un pôle d'électrode (30, 32) relié avec le conducteur fonctionnel par l'intermédiaire duquel le courant électrique peut être délivré dans le tissu corporel environnant en cas de besoin ou avec lequel des potentiels électriques peuvent être appliqués dans le tissu environnant en cas de besoin, ou les deux, l'appareil médical présentant au moins un composant électrique qui se trouve à l'intérieur du boîtier (12, 100) et dont la longueur électrique correspond à au moins un quart de la longueur d'onde λ d'ondes électromagnétiques dans le domaine des fréquences radio qui se produisent dans un appareil de résonance magnétique nucléaire pour 1,5 T ou 3 T, où le conducteur fonctionnel est un conducteur électrique (26) d'une ligne d'électrode (20, 102) qui présente au moins un pôle d'électrode (30 ; 32), lequel est relié électriquement avec les composants électriques disposés à l'intérieur du boitier (12, 100) dont la longueur électrique correspond à au moins un quart de la longueur d'onde λ d'ondes électromagnétiques dans le domaine des fréquences radio qui se produisent dans un appareil de résonance magnétique nucléaire pour 1,5 T ou 3 T.

2. Appareil médical selon la revendication 1, **caractérisé en ce que** l'appareil médical possède un boîtier (12, 100), lequel est conçu conducteur électriquement ou présente un pôle d'électrode (30, 32) par l'intermédiaire duquel du courant électrique peut être délivré dans du tissu corporel environnant en cas de besoin, ou avec lequel des potentiels électriques peuvent être appliqués dans du tissu environnant, ou les deux.

3. Appareil médical selon au moins une des revendications 1 ou 2, **caractérisé en ce que** l'appareil médical présente un composant (130) pour l'ajustement de l'impédance entre le conducteur fonctionnel (110) et les composants (140) conducteurs électriquement avec une longueur électrique > λ/4, qui est prévu pour ajuster des impédances du conducteur fonctionnel (110) et des composants (140) électriquement conducteurs avec la longueur électrique > λ/4.

4. Appareil médical selon la revendication 3, **caractérisé en ce que** le composant d'ajustement d'impédance (130) est un bras de réactance ou présente un tel bras de réactance.

5. Appareil médical selon la revendication 3 ou 4, **caractérisé en ce que** le composant est un symétriseur pour l'ajustement d'impédance (130) ou présente un tel symétriseur.

6. Appareil médical selon au moins une des revendications 3 à 5, **caractérisé en ce que** le composant (130) pour l'ajustement d'impédance est un condensateur (134) ou présente un tel condensateur.

7. Appareil médical selon au moins une des revendications 3 à 6, **caractérisé en ce que** le composant (130) pour l'ajustement d'impédance est une inductance ou présente une telle inductance.

8. Appareil médical selon au moins une des revendications 3 à 7, **caractérisé en ce que** le composant (130) pour l'ajustement d'impédance est une combinaison d'une inductance et d'un condensateur ou présente une telle combinaison.

9. Appareil médical selon au moins une des revendications 1 à 8, **caractérisé en ce que** le composant électrique (140), dont la longueur électrique correspond à au moins un quart de la longueur d'onde λ des ondes électromagnétiques dans le domaine des fréquences radio, est isolé sur sa longueur.

10. Appareil médical selon au moins une des revendications 1 à 9, **caractérisé en ce que** le composant électrique (140) dont la longueur électrique correspond à au moins un quart de la longueur d'onde λ des ondes électromagnétiques dans le domaine des fréquences radio a un contact avec un milieu qui est constitué totalement ou par endroits des matériaux suivants ou d'une combinaison des matériaux suivants : le verre, le titanate de baryum, le polyéthylène, l'oxyde de silicium, le carbure de silicium, la céramique, les matières piézoélectriques, l'oxyde d'aluminium, l'oxyde de titane, l'oxyde de tantale, les matériaux semi-conducteurs, les matières synthétiques avec des mélanges des matériaux cités ci-dessus.

11. Appareil médical selon au moins une des revendications 1 à 10, **caractérisé en ce que** le composant électrique (140), dont la longueur électrique correspond à au moins un quart de la longueur d'onde λ des ondes électromagnétiques dans le domaine des fréquences radio, présente un conducteur d'extension (142) qui est réalisé sous la forme de l'une des configurations ou d'une combinaison : de fil et d'une bande, de fil et d'une bande platinés, de fil et d'une bande enroulés, d'un toron, d'une corde, d'un conducteur imprimé, d'un conducteur coupé, d'un conducteur gravé, d'un conducteur de dépôt galvanique, d'une surface conductrice fendue.
